# EUROPEAN PATENT APPLICATION

(11) **EP 4 527 940 A1**
(43) Date of publication of application: **26.03.2025**
(21) Application number: 23198397.4
(22) Date of filing: 19.09.2023
(51) Int. Cl.: C12P 33/06, C12N 9/02

(54) **NEW PROCESS FOR PRODUCTION OF CALCIFEDIOL**

(71) Applicant: DSM IP Assets B.V., 6221 BE Maastricht (NL)
(72) Inventor: May, Oliver, 4303 Kaiseraugst (CH); Willistein, Max, 79098 Freburg (DE)
(74) Representative: dsm-firmenich IP

(57) **Abstract**

The present invention relates to an improved process for production of calcifediol via biotransformation of cholecalciferol. The biologically inactive calcifediol is the major circulating and storage form of vitamin D and thus an important composition in the food and pharma sector. The novel process enables recycling of necessary components including the biocatalyst.

## Description

The present invention relates to an improved process for production of calcifediol via biotransformation of cholecalciferol. The biologically inactive calcifediol is the major circulating and storage form of vitamin D and thus an important composition in the food and pharma sector. The novel process enables recycling of necessary components including the biocatalyst.

Vitamin D3 (cholecalciferol) is a very unique vitamin that is synthesized in the skin of mammals via exposure to UV light from 7-dehydrocholesterol (7-DHC), a product from the cholesterol biosynthesis. In the liver, said cholecalciferol is converted into the biologically inactive calcifediol (also known as 25-hydroxycholecalciferol, 25-hydroxyvitamin D3, calcidiol, 25-OH-D3 or 25-HyD), which is the major circulating and storage form of vitamin D. Further hydroxylation to calcitriol (also I<nown as 1α,25-dihydroxy-vitamin D3) occurs in the kidney. Calcitriol can bind to specific steroid hormone receptors to regulate transcription of certain vitamin D sensitive genes.

Chemical production of vitamin D3 including the hydroxylated forms typically starts from cholesterol isolated from e.g. wool fat which is dehydrogenated into 7-DHC. Through exposure by UV-light and further purification/extraction steps 7-DHC is converted into cholecalciferol and/or further into calcifediol. Such highly complex processes are not very sustainable due to high consumption of resources and energy.

For bioconversion of cholecalciferol into biologically inactive hydroxylated vitamin D3 forms, members of the cytochrome P-450 superfamily such as e.g. isolated from Pseudomonas autotrophica have been described, however these enzymes are very unspecific with regards to hydroxylation at a certain position. Thus, mutations had been introduced to increase selectivity towards C-25 hydroxylation (Yasutal<e et al., ChemBioChem 14, 2284-2291, 2013).

Some enzymes of the dimethylsulfoxide reductase (DSMOR) family of molybdenum cofactor-containing enzymes are known to regio-selectively catalyze hydroxylation of the 25-position, such as e.g. steroid C25 dehydrogenase (S25DH) isolated from *Sterolibacterium denitrificans.* A substrate conversion of 92% after 16 h could be achieved using crude extracts from S. denitrificans anaerobically grown with cholesterol in the presence of 2-hydroxypropyl-β-cyclodextrin (HPCD) and K₃[Fe(CN)₆] as regenerative cofactor (Warnl<e et al., Angew. Chem. 2016, 128, 1913-1916).

An expression system in the denitrifying bacterium *Thauera aromatica* was established to express O2-sensitive recombinant S25DH enzymes. Yields of over 95% could be achieved using crude extracts from *T. aromatica* expressing S25DH. Heterologous expression in more characterized host systems like *E. coli* however failed (Jacoby et al., MBio 9(3), 300694-18, 1-14, 2018).

WO2022101282 discloses a process with recombinant S25DH expressed with *T*. *aromatica,* wherein resting cells are used together with nitrate as terminal electron acceptor under anaerobic conditions. A dropping of the initial high conversion rates was detected, mostly due to consumption of nitrate for cell metabolism, leading to formation of nitrite as non-desirable side-product.

While the use of cyclodextrins as carrier molecules for vitamin D3 or 7-DHC has shown to be helpful, their high cost limits their industrial application. In addition, it is known that cyclodextrins can be degraded via spontaneous or enzyme catalyzed reactions (Munteanu & Ritter, chapter 16, in: Biocatalysis in Polymer Chemistry, Editor: Loos, Katja, 2011, chapter 16, 389-420, Wiley-VCH Verlag GmbH & Co. KGaA; DOI: 10.1002/9783527632534.ch16). This could further limit their use by increasing costs as part of the cyclodextrin is lost during the transformation and requires costly purification steps to remove degradation products.

Thus, there is still a need for optimizing the biotechnological production of vitamin D3 metabolites, particularly towards an industrial applicable, sustainable, cost-efficient and regio-selective biotransformation process leading to C-25 hydroxylated vitamin D3 metabolites.

Surprisingly, we now found a way to establish a sustainable process using whole cells, said process comprising recycling of the biocatalyst, without compromising the enzyme activity or product yield. Additionally, we found a way to reuse the required solubilizer, such as particularly cyclodextrin, with a minimization of its degradation during such process.

Thus, the present invention relates to the production of C-25 hydroxylated vitamin D3 metabolites, comprising contacting a non-hydroxylated form of vitamin D3 with a biocatalyst capable of C-25 selective hydroxylation in the presence of a solubilizing agent, particularly cyclodextrin, such that C-25 hydroxylated metabolites such as e.g. 25-HyDHC or 25-HyD are formed, isolating the biocatalyst and optionally cyclodextrin from the other compounds, wherein the contacting and the isolating steps are repeated for at least 2 to 3 times. Using the biocatalyst and/or cyclodextrin for at least 2 to 3 times, the activity towards formation of C-25 hydroxylated vitamin D3 metabolites is not decreased nor is the cyclodextrin degraded to an extent that limits its function as a carrier.

The term "vitamin D3" as used herein includes vitamin D3 and intermediates or metabolites thereof as known in the art, both in hydroxylated or non-hydroxylated form. Examples of vitamin D3 intermediates or metabolites include but are not limited to C25-hydroxyvitamin D3 (25-HyD), 1α,25-dihydroxyvitamin D3, 1α-hydroxyvitamin D3, 7-DHC, 24R,25-dihydroxycholecalciferol, 25-HyDHC (see Bendil< et al., Ullmann's Encyclopedia of Industrial Chemistry, 2019, Wiley-VCH).

As used herein, the term "hydroxylated vitamin D3" particularly includes C-25 hydroxylated metabolites such as 25-HyDHC or 25-HyD (also referred herein as HyD).

The terms "regio-selective" and "selective" in connection with vitamin D3 hydroxylating enzymes as defined herein are used interchangeably.

The terms "conversion", "hydroxylation" or "enzymatic conversion" are used interchangeably herein and describe the hydroxylation of vitamin D3 or derivatives such as e.g. 7-DHC leading to a mix of mono- and di-hydroxylated vitamin D3 derivatives including but not limited to HyD or HyDHC. The term "biotransformation" and "enzymatic conversion" is used interchangeably herein and describes the transformation of compound A into compound B catalyzed by an enzyme, such as e.g. transformation of 7-DHC into 25-HyDHC or vitamin D3 into HyD catalyzed by regio selective C25-dehydrogenase, oxygenases or peroxygenases.

As used herein, a "solubilizing agent" refers to an agent helping to increase the solubility of other compounds, such as e.g. steroids like vitamin D3 and derivatives. An example of a useful solubilizer according to the present invention is cyclodextrin, preferably HPCD, and optionally further comprising cosolvents, such as e.g. ethanol or isopropanol.

The present invention leads to a process wherein the used cyclodextrin is not degraded to an extent that limits its function as a carrier. Thus, the process as defined herein comprising particularly at least 2 to 3 cycles leads to degration of cyclodextrin, particularly HPCD in the range of 20% or less, preferably degradation in the range of 15, 10, 8, 7, 5, 4, 3, 2, 1% or less.

In some embodiments, the biotransformation process comprises HPCD concentrations of up to 15%% (w/v), such as in the range of 15-1% (w/v), i.e. 14.5, 14, 13.5, 13, 12.5, 12, 11.5, 11, 10.5, 10, 9.5, 9, 8.5, 8, 7.5, 7, 6.5, 6, 5.5, 5, 4.5, 4, 3.5, 3, 2.5, 2, 1.5, 1% (w/v), preferably concentrations in the range of 10 to 12.5% (w/v), more preferably 2.5 to 7.5% (w/v).

In some embodiments, the biotransformation process comprises the use of cosolvents selected from isopropanol or ethanol, preferably with concentrations of 1-5% (v/v) isopropanol or ethanol.

Thus, the present invention is particularly directed to C-25 selective hydroxylation of vitamin D3 in the presence of HPCD, preferably with EtOH as co-solvent.

Suitable enzymes for C-25 selective hydroxylation might be selected from members of oxygenases such as P450 monooxygenases, peroxygenases or the iron sulfur molybdoenzyme family, particularly steroid C25 dehydrogenases (S25DHs), more particularly isoforms of S25DH isolated from Sterolibacterium denitrificans (Jacoby et al. (MBio 9(3), 300694-18, 1-14, 2018). Preferably, the enzymes used for the present invention are expressed as heterologous enzymes in a suitable host cell, such as e.g. using an inducible expression plasmid or integrated into the host cell's genome. The enzymes are particularly used in the form of cells, e.g. whole cells, containing said C-25 selective enzymes or as cell extract.

Using the biocatalyst and/or cyclodextrin for at least 2 to 3 times, the activity towards formation of C-25 hydroxylated vitamin D3 metabolites is not decreased nor is the cyclodextrin degraded to an extent that limits its function as a carrier.

According to the present invention, the biocatalyst, i.e. a C-25 selective enzyme as defined herein, is to be re-used for at least 2 to 3 times without a "decrease in the ability of the biocatalyst towards C-25 hydroxylation". Thus, the capability of the enzyme for C-25 selective hydroxylation is maintained at more or less the same level, with a maximum reduction of hydroxylation in the range of 5% or less in a multiple cycle process, such as e.g. re-use of the biocatalyst for at least 2 to 3 cycles, wherein the reduction of hydroxylation of the substrate as definded herein is in total 5% or less, such as 4, 3, 2, 1% or even less.

Suitable host cells are selected from denitrifying bacteria, particularly denitrifying proteobacteria, including but not limited to species of the genera *Thauera,* preferably *T. aromatica* , *Aromatoleum,* preferably *A*. *aromatoleum, Azoarcus,* preferably A. *evansii, Sterolibacterium,* preferably *S*. *denitrificans,* or even *E. coli or yeast such* as *e.g. Saccharomyces, particularly S. cerevisiae,* using a suitable expression system, such as e.g. disclosed by Jacoby et al. (MBio 9(3), 300694-18, 1-14, 2018).

The host cell expressing the enzymes as defined herein may be cultured under aerobic, semi-aerobic or anaerobic conditions in the presence of a suitable carbon source and nutrients as known in the art or as described in e.g. WO2022101282.

Particularly, cultivation is conducted under semi-aerobic conditions with induction of expression of the C-25 selective enzymes as defined herein, particularly S25DH expression, in the exponential growth phase.

In some embodiments, cultivation under semi-aerobic conditions as defined herein is conducted for at least about 150 h and up to about 200 h, preferably 160 to 190h at 30°C, leading to a high-density cell suspension.

As used herein, the term "high-density cell suspension" means suitable host cells expressing the C25-selective enzymes including S25DH as defined herein grown under conditions as described herein until a cell density of at least OD=5 is reached. Cells are harvested by centrifugation and used for the biotransformation, either as cell extract or whole cells containing said C25-selective enzymes as defined herein.

As used herein, the term "cell extract" means that the cells are disrupted at the end of cultivation to generate the extract, such as e.g. via application of sonication or French press. It includes both viable and non-viable cells.

As used herein, the term "semi-aerobic" means that the oxygen is not actively removed from the medium and feed stocks to maintain the denitrifying conditions due to consumption of initially dissolved oxygen by e.g. constitutively produced oxidases.

As used herein, the term "anaerobic" means active removal of oxygen from the medium and the feed stocks to maintain the denitrifying conditions.

Suitable carbon sources to be used for the present invention might be selected from acetate, particularly sodium acetate or acetic acid.

In sone embodiments, the host cell expressing heterologous C-25 selective enzymes including S25DH is selected from *T. aromatica* and grown under semi-aerobic conditions with acetate as carbon source until an OD of 3-5, such as cultivated for 160-190 h at 30°C.

In some embodiments, the C-25-selective enzymes including S25DH as defined herein might be expressed with an arabinose or lactose-inducible expression system as known in the art (see e.g. Guzman et al., 1995, J. Bacteriol., 177, 4121-4130).

In some embodiments, the biotransformation is performed using the substrate, such as e.g. vitamin D3 or 7-DHC, solubilized in HPCD and EtOH as co-solvent, said substrate being contacted with a high-density cell suspension as described above, either as whole cells or cell extract, preferably as whole cells, wherein the biotransformation is preferably conducted under anaerobic conditions with K₃[Fe(CN)₆] as co-factor.

In particular embodiments, the biotransformation is performed under anaerobic conditions with concentration of the C-25 selective biocatalyst, particularly S25DH, in the range of 20g/L and a concentration of substrate, such as e.g. vitamin D3 or 7-DHC, in the presence of about 10% HPCD, in the range of 1 g/L wherein a yield of 65-99% is achieved after 22-24h. The biocatalyst might be present in the form of whole cells or cell extract.

In particular embodiments, an extract of T. aromatica cells expressing S25DH with 20.5 g/l biocatalyst is contacted with 0.98g/L vitamin D3 in the presence of 100 g/L HPCD, 5% v/v EtOH and 3.39 g/l FeCN for 22 h, resulting in a yield of 99.2%, with a maximum of 1.24 g/L HyD obtained for 22h under anaerobic conditions with FeCN as co-factor.

In particular embodiments, the biotransformation reaction is performed under conditions comprising the presence of 5% (w/v) HPCD and 1-5% (v/v) isopropanol together with 0.5 mM of substrate such as e.g. vitamin D3 or 7-DHC wherein an extract of *T. aromatica* expressing S25DH1 (OD=100) is used.

In particular embodiments, the biotransformation reaction is performed under conditions comprising the presence of 10% (w/v) HPCD and 5% (v/v) ethanol together with 2.5 mM of substrate such as e.g. vitamin D3 or 7-DHC, wherein a cell suspension of resting cells of *T. aromatica* expressing S25DH1 (OD=100) is used.

Cultivation time might vary, e.g. 160 to 190h at 30°C depending on the host cell. Hydroxylated vitamin D3 derivatives including HyD might be isolated and/or optionally further purified and used as ingredient in respective formulations in food, feed, cosmetic or pharma sector.

In some embodiments, the hydroxylation/biotransformation of vitamin D3 or derivatives such as e.g. 7-DHC as defined herein is performed via contacting an extract of *T. aromatica* heterologously expressing a C-25 selective enzyme, such as e.g. steroid C25 dehydroxylase as defined herein with the substrate under anaerobic conditions described herein, said conversion being conducted in the presence of 10% (w/v) HPCD and 5% EtOH, wherein the cell stability during the conversion is maintained for at least about 22h.

The present invention is directed to bioproduction of C-25 hydroxylated vitamin D3 metabolites as defined herein, comprising recycling of the biocatalyst, preferably wherein the biocatalyst is used in the form of whole cells.

In some embodiments, the biotransformation according to the present invention comprises recycling of the biocatalyst with at least 3 consecutive 22 h cycles at 30°C, wherein the conversion rates and total product yield is not reduced or stays at a constant level over consecutive cycles. In some preferred embodiments, the product yield might increase over the consecutive cycles.

For the purpose of the present invention, recycling of cells comprising the biocatalyst means that after the end of conversion/hydroxylation of the substrate as defined herein the cells are removed from the conversion medium by centrifugation and again mixed with vitamin D3 or 7-DHC (i.e. the substrate) in the presence of HPCD and a co-solvent such as EtOH for another cycle of biotransformation.

In one particular embodiment, the present invention is directed to a bioproduction of 25-hydroxylated vitamin D3 metabolites, particularly comprising 25-HyDHC and HyD, wherein a substrate, such as vitamin D3 or 7-DHC, in the presence of a solubilizing agent, particularly cyclodextrin, more particularly HPCD, is hydroxylated at C-25 through the catalytic activity of S25DH, particularly S25DH originated from *S*. *denitrificans,* said enzyme being heterologously expressed in a suitable host cell, particularly selected from *Thaurea,* preferably *T. aromatica,* wherein the biocatalyst is used in the form of whole cells or a cell extract and wherein the biotransformation is performed under oxygen limitation at 30°C and wherein the above-described biotransformation cycle is repeated for at least 2-3 times.

In one particular embodiment, the present invention is directed to a biotransformation process as defined herein comprising 3 consecutive cycles of conversion reaction wherein the whole cell catalyst is recycled via centrifugation after each cycle followed by mixing with fresh substrate for the following cycle, particularly with each cycle running for 22h, wherein the yield could be maintained or even increased with every cycle and wherein the conversion and the biocatalyst recycling is conducted under anaerobic conditions, particularly wherein the biotransformation is conducted in the presence of K₃[Fe(CN)₆] and 5-10% (w/v) HPCD and 5-10% EtOH.

The present invention is particularly directed to a multi-cycle anaerobic biotransformation reaction leading to HyD or 25-OH-7-DHC as defined herein, wherein the yield can be increased with each cycle for at least about 5,10,15, 20, 25, 30, 35, 40, 45, 50% compared to the previous cycle.

In some embodiments of the present invention, C-25 hydroxylated vitamin D3 metabolites are formed in a biotransformation process as defined herein, comprising (quantitative) recycling of the solubilizer HPCD, wherein after biotransformation and removal of the cells via centrifugation to be re-used for another cyle of biotransformation, the remaining aqueous phase is further extracted leading to a reusable solution of HPCD and buffer components. Particularly, recycling of HPCD from whole cell conversion mixtures over 10 cycles could be achieved, with no negative impact on quality or quantity of HPCD.

As used herein, the term "specific activity" or "activity" with regards to an enzyme means its catalytic activity, i.e. its ability to catalyze formation of a product, i.e. hydroxylated vitamin D3 derivatives comprising HyD or HyDHC, from a given substrate, i.e. vitamin D3 or 7-DHC. The specific activity defines the amount of substrate consumed and/or product produced in a given time period and per defined amount of protein at a defined temperature. Typically, specific activity is expressed in µmol substrate consumed or product formed per min per mg of protein. Typically, activity is given per mg of the specific enzyme; if specified otherwise, activity can also be given on mg enzyme preparation (e.g. total protein, total soluble protein etc.). Typically, µmol/min is abbreviated by U (= unit). Therefore, the unit definitions for specific activity of µmol/min/(mg of protein) or U/(mg of protein) are used interchangeably throughout this document. An enzyme is active, if it performs its catalytic activity *in vivo,* i.e. within the host cell as defined herein or within an *in vitro* system in the presence of a suitable substrate. The skilled person knows how to measure enzyme activity, in particular activity of C-25 selective enzymes including S25DHs as defined herein, such as in particular C-25 hydroxylation activity as defined herein. Analytical methods to evaluate the capability of a suitable enzyme as defined herein for formation of C-25 hydroxylated vitamin D3 metabolites as defined herein are known in the art.

With regards to the present invention, it is understood that organisms, such as e.g. animals, microorganisms, fungi, algae or plants also include synonyms or basonyms of such species having the same physiological properties, as defined by the International Code of Zoological Nomenclature, International Code of Nomenclature of Prokaryotes, the International Code of Nomenclature for algae, fungi, and plants (Melbourne Code), or the International Commission of the Taxonomy of Fungi (ICTF).

### Figures

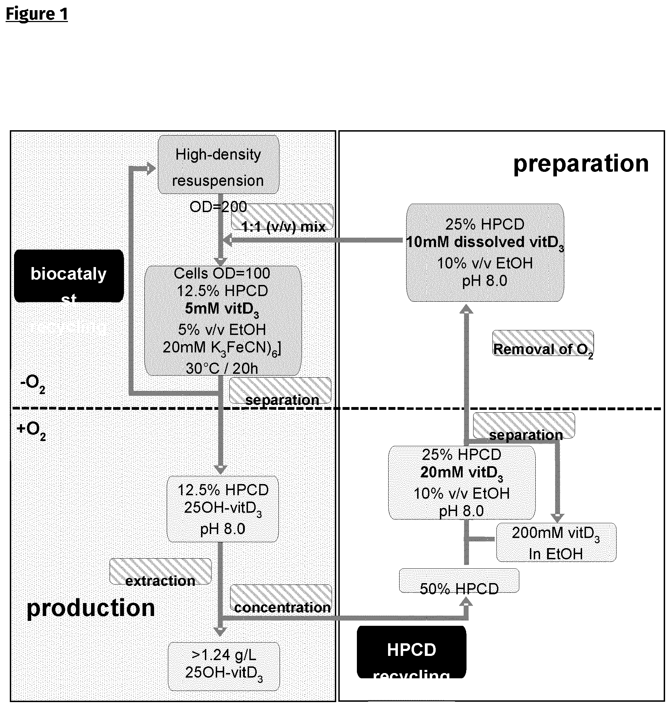

**Figure 1****.** Workflow for bioconversion of vitamin D3 indicating the semi-aerobic or anaerobic conditions ("+O2"; steps marked with dark grey colored boxes) and aerobic conditions ("-O2"; steps marked with light grey colored boxes) of the process including different steps involved in preparation of cells, substrate and HPCD as solublizer as well as the steps involved in production of HyD ("250H-vitD3") and HPCD. Whereas recycling of the biocatalyst is conducted at semi-aerobic/anaerobic conditions, recycling of HPCD is conducted at aerobic conditions.

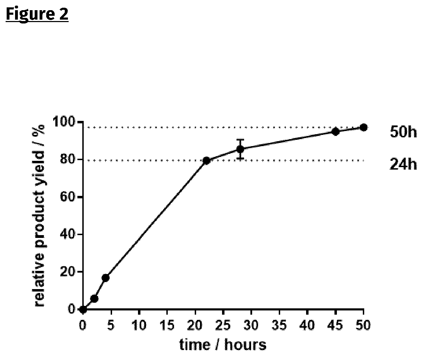

**Figure 2****.** Bioconversion of vitamin D3 using whole cells of *T*. *aromatica* expressing S25DH grown on acetate/nitrate until OD=100. The time [hours] is shown on the x-axes, relative product yield [%] is shown on the y-axes. For further details, see text.

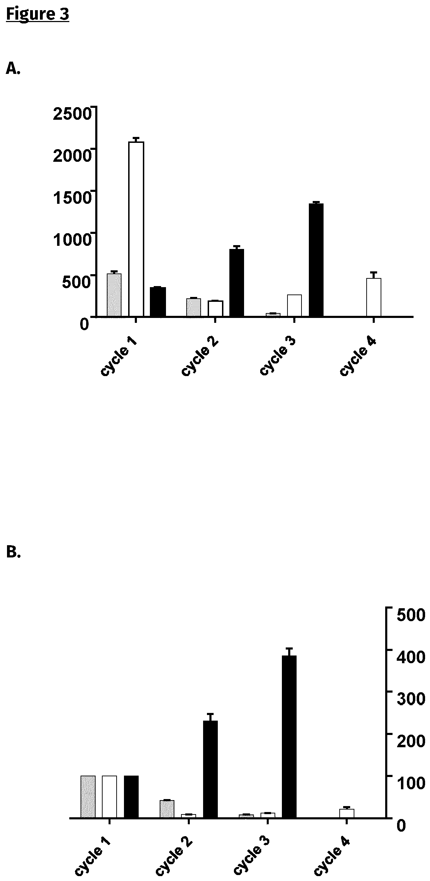

**Figure 3****.** Conversion rate and product yield using recycled cells. The total HyD [µM] produced in 22h using cells of OD=100 **(****Fig.3A****)** and the relative product yield [%] **(****Fig.3B****)** as shown on the y-axes is given for different incubation conditions and electron acceptors: aerobic / oxygen (grey), anaerobic / nitrate (white), anaerobic / K₃[Fe(CN)₆] (black) as shown on the x-axes. Cells were recycled by centrifugation before fresh substrate was added for the following cycle. The aerobic recycling contained 0.5 mM vitamin D3, 5% w/v HPCD and 1% v/v isopropanol while the anaerobic approaches contained 5 mM VitD3, 20% w/v HPCD and 5-10% EtOH. For further details, see text.

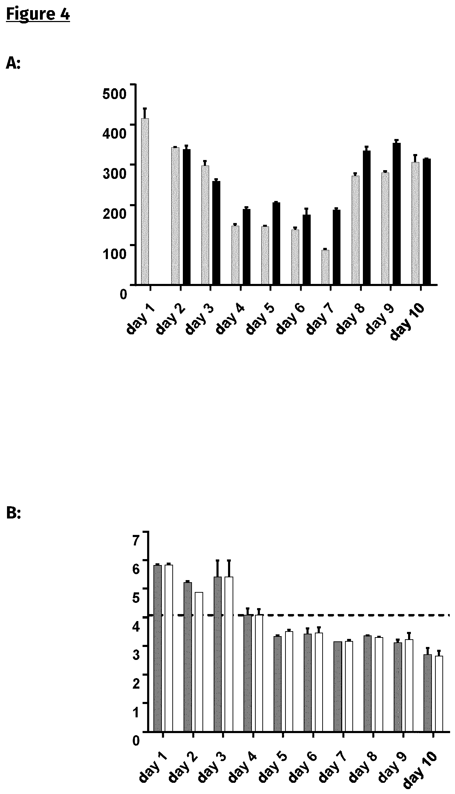

**Figure 4****.** Results from HPCD recycling over 10 days shown on the x-axes/conversions. **Fig.4A** shows formation of HyD [µM] in 4h using cells of OD=100 (y-axes) produced during 10 consecutive cycles/days with recycled HPCD (grey) or a control experiment with freshly added HPCD (black). **Fig.4B** shows change in HPCD concentration (% w/v) on the y-axes in recycled solutions for VitD3 oxidation. The HPCD concentration was determined before the start of a new conversion cycle (dark grey) and after the cycle was finished (white) before extraction of residual substrate and product. The minimum expected total loss due to sampling after 10 cycles is indicated by a dotted line. For further details, see text.

The following examples are illustrative only and are not intended to limit the scope of the invention in any way.

### Examples

### Example 1: General methods, media and cultivation

All basic molecular biology and DNA manipulation procedures described herein were generally performed according to Sambrook et al. (1989. Molecular Cloning: A Laboratory Manual. Cold Spring Harbor Laboratory Press: New York) or Ausubel et al. (1998. Current Protocols in Molecular Biology. Wiley: New York).

Cultivation of Thauera aromatica: T. aromatica K172 (DSM 6984) was cultivated under semi-aerobic conditions (no active removal of oxygen) in mineral salt medium (Table 1) and basically as described in WO2022101282 (see Examples) with cultivation at pH8 at 30°C without shaking. The base medium (MSM; without additives) and all stock solutions were prepared under aerobic conditions (exept vitamins and trace elements to prevent degradation during storage), as cultures tolerate limited oxygen influx while still maintaining denitrifying conditions during static incubation. Gentamicin was added to a final concentration of 50 µg/mL to ensure plasmid stability.

Optical density determination at 578 nm was used to follow the progress of cell cultivation. Samples of 1 mL were measured in semi micro-cuvettes in a spectrophotometer. At OD > 0.4, samples were diluted 1:10 v/v with MSM. MSM was used as a blank. Nitrate consumption was monitored using semiquantitative teststrips (quantofix nitrate/nitrite II, Macherey-Nagel, Düren, Germany). Changes in pH were monitored by pH-strips (pH-Fix 4.5-10, Roth, Karlsruhe, Germany).

Media used for protein production are listed in Table 1.

**Table 1: Media composition.**

| Medium | Components |
|---|---|
| MSM (base medium) | 0.69 g/L NaH₂PO₄ × 1 H₂O, |
| | 7.3 g/L K₂HPO₄ × 3 H₂O |
| | 0.54 g/L NH₄Cl |
| | medium adjusted to pH=8 with NaOH before sterilization to be added from separate stock: |
| | 0.20 g/L MgCl₂ × 6 H₂O |
| | 0.02 g/L CaCl₂ × 2 H₂O |
| | 1.28 g/L NaNO₃ |
| | 2.04 g/L NaOAc × 3 H₂O |
| VL-7 (prepared as 1000x concentrated stock; sterilized) | 200 mg/L nicotinic acid |
| | 20 mg/L D(+)-biotin |
| | 20 mg/L thiamine hydrochloride |
| | 80 mg/L 4-aminobenzoic acid |
| | 100 mg/L Ca-D(+)-pantothenate |
| | 300 mg/L pyridoxine-dihydrochloride |
| | 100 mg/L cyanocobalamine |
| SL-10 (prepared as 1000x concentrated stock; sterilized) | 1.5 g/L FeCl₂ × 4 H₂O |
| | 70 mg/L ZnCl₂ |
| | 100 mg/L MnCl₂ × 4 H₂O |
| | 2 mg/L CuCl₂ × 2 H₂O |
| | 190 mg/L CoCl₂ × 6 H₂O |
| | 24 mg/L NiCl₂ × 6 H₂O |
| | 36 mg/L Na₂MoO₄ × 2 H₂O |
| | 6 mg/L H₃BO₃ |
| | 143.3 g/L NTA |
| | 58 g/L NaOH |

Preparation of glycerol stock cultures for storage: For long-term storage, 100 mL of MSM (+additives) containing 15 mM of sodium acetate and 15 mM sodium nitrate were inoculated with a growing culture (1:100 v/v) and incubated for 48h at 30°C to optical densities of up to OD578 = 1.0. Small portions of the liquid culture (1 mL) were then mixed with 1 mL of 50% w/v sterile glycerol (final concentration 25% w/v) and shock frozen in liquid nitrogen. The stock cultures were then stored at -80°C.

Preparatory cultures: For preparatory cultures, 100 mL of MSM (+additives) containing 15 mM sodium acetate and 15 mM sodium nitrate were inoculated with a glycerol stock culture (2 mL) and incubated for 48h at 30°C. The preparatory culture was then used for larger-scale cultivation.

Cultivation for S25DH production: For heterologous S25DH production, 1.0-1.8 L of MSM (+additives) containing 15 mM sodium acetate and 15 mM sodium acetate were inoculated with a preparatory culture (1:50 to 1:100 v/v) and incubated for up to 180h at 30°C. Nitrate consumption and pH were checked twice every day to adjust pH to 8.0 and supply sodium acetate/acetic acid (depending on pH) and sodium nitrate in 15 mM portions when nitrate was consumed. When the cultures reached the stationary growth phase at OD578 = 3-5, no more nutrients were added and the cultures were stored at 4°C.

Preparation of premixed vitamin D3 solutions: premixed solutions oversaturated with vitamin D3 were prepared. A solution of 50% HPCD (w/v) in ddH₂O was prepared and mixed with the required volume of 100-200 mM vitamin D3 stock solution in EtOH to obtain a final concentration of 10-20 mM of vitamin D3 under constant stirring. The mixture was diluted with MSM to the desired final volume. Approximately 50% of the added vitamin D3 precipitated and was removed by filtration (0.2 µm filter) to obtain a clear solution. Typical concentrations of 6-10 mM dissolved vitamin D3 were obtained in the presence of 20-25% HPCD and 1-5% v/v EtOH as co-solvent.

Analysis of protein production in whole cells: For analysis of protein production in whole cells, 250 µL of cell culture at OD578 = 1.0 or an equivalent volume at different densities was centrifuged at 14000 rpm and 4°C for 15 min. The pellet was then resuspended in 35 µL of sample buffer (100 mM TRIS/HCl pH 6.8, 4% w/v sodium dodecylsulfate, 20% w/v glycerol, 200 mM dithioerythritol, 0.1% w/v bromophenol blue) and incubated at 95°C for 10 min. The sample was then centrifuged at 14000 rpm at room temperature for 10 min and 20 µL of the supernatant were analysed by SDS-PAGE.

UPLC analysis: For UPLC analysis, samples were taken at defined times and added to a 4x excess volume of isopropanol to stop the reaction (20 µL sample + 80 µL isopropanol). The samples were then centrifuged (20 min, RT, 14000 rpm) and the supernatant was centrifuged again (20 min, RT, 14000 rpm).

Samples (5 µL) were then separated by reversed-phase chromatography (Waters Acquity UPLC CSH C18 column, 2.1 × 100 mm, 1.7 µm particle size) on a Water H-Class UPLC system (Waters, Milford, USA) using acetonitrile + 0.1% v/v formic acid / ddH₂O + 0.1% fo00rmic acid as eluent at a flow rate of 0.4 mL/min at 50°C column temperature. Samples were applied at 50% v/v organic solvent and eluted at 100% ACN + 0.1% formic acid (isocratic, 4 min). Analytes were detected by absorption at 260 nm (Waters UV/vis diode array). For determination of absolute vitamin D3 and 25-hydroxy-vitamin D3 (HyD) concentrations, peak areas were calculated at 260 nm and compared to calibration curves prepared with authentic standards (Sigma-Aldrich, Taufl<irchen, Germany).

HPCD determination: The photometric detection of hydroxypropyl-β-cyclodextrin (HPCD) was modified from Goel and Nene (Starch 47(10): 399-400, 1995) and Mäkelä et al. (J Biochem Biophys Methods 14(2): 85-92, 1987) and is based on the reduction of phenolphthalein absorption after complexation with cyclodextrins.

A working solution was prepared by mixing 20 mL 125 mM NaHCO₃ solution with 800 µL EtOH and 200 µL of a 4 mM solution of phenolphthalein in EtOH.

Clarified samples (centrifugation at 14000 rpm, room temperature, 20 min) were diluted with 50 mM TRIS/HCl pH 7.0 to an HPCD concentration of 0-100 µg/mL. The diluted samples (200 µL) were then mixed with 800 µL of working solution. The absorption at 550 nm was immediately measured after addition of working solution. For quantification, samples that contained 0-50 µg/mL of HPCD were used to prepare a calibration curve. The HPCD standards (200 µL) were mixed with working solution (800 µL) immediately before the absorbance was measured at 550 nm. The standard containing no HPCD was used as a blank.

Expression system of S25DH: The genes of the biocatalyst as used in the present invention were cloned into the broad-host-range vector pIZ1016 (Gmr, oripBBR1, Mob+, lacZa, Ptac/lacIq) and transformed into Thauera aromatica K172 as described (Jacoby et al., MBio 9(3), 300694-18, 1-14, 2018; Fig. 5 of WO2022101282). For conversion of vitamin D3, S25DH1 is best performing isoform, best conversion of 7-DHC can be achieved with S25DH2 (Jacoby et al., MBio 9(3), 300694-18, 1-14, 2018).

### Example 2: Conversion of vitamin D3 by whole cell biocatalyst

Cultivation of T. aromatic strains expressing heterologous S25DH isoforms ware prepared as described above. All steps were carried out with anaerobic buffers and solutions (N₂) in an anaerobic atmosphere (N₂/H₂ 95:5 v/v).

T. aromatica cells after production of S25DH1 were harvested by centrifugation in 50 mL conical tubes (6000 rpm, 4°C, 20 min, Eppendorf 5804R centrifuge, Rotor S 4 72). When complete removal of residual cultivation medium was required, the cell pellet was washed by resuspension in fresh MSM followed by centrifugation (6000 rpm, 4°C, 20 min).

Cell pellets were resuspended in the appropriate amount of MSM to obtain a suspension with an optical density of OD578 = 200. The cell suspension was then mixed in a 1:1 ratio (v/v) with a premixed solution of VitD3, EtOH and HPCD in MSM as described above and incubated at 30°C shaking at 500 rpm (for reactions in test tubes) or stirring at 500 rpm (see Figure 1).

After bioconversion, cells were removed by centrifugation (6000 rpm, 4°C, 20 min) and used for further cycles. The clear aqueous solution was then extracted three times with a three times excess volume of ethyl acetate. HPCD was removed and used for further cycles, whereby HPCD and buffer substances are retained in the aqueous fraction and used again to dissolve fresh vitamin D3 and the electron acceptor and to resuspend fresh cells for consecutive cycles. The combined organic phases were then analysed by UPLC after dilution in isopropanol as described above.

As shown in Figure 2, a max. yield of 97% HyD after 50h starting from OD = 100 was obtained, wherein the cells were grown on acetate/nitrate, in the presence of 12.5 w/v HPCD, 2.5 v/v EtOH and totally 50 mM K₃[Fe(CN)₆] (in portions of 10 mM) as electron acceptor.

### Example 3: Recycling experiments

Recycling of whole cell catalyst was evaluated under various conditions, both under aerobic and anaerobic conditions, wherein the artificial electron acceptor K₃[Fe(CN)₆] was tested against nitrate. Cells were resuspended to OD=100 for initial cycles and then recycled by centrifugation before fresh substrate (0.5 mM vitamin D3) was added for the following cycle. The results are depicted in Figure 3, showing that with the use of recycled of cells under anaerobic conditions in the presence of K₃[Fe(CN)₆] the yield could be massively increased with the number of cycles, at least with 3 cycles.

Since the solubilizing agent such as HPCD is essential for mediation of solubility of vitamin D3, experiments were performed aiming at the recycling of the solubilizer. Thus, parallel setups were conducted using either freshly prepared HPCD or recycled HPCD, wherein 10 cycles/bioconversions were run. For using the recycled HPCD, cells were removed by centrifugation or filtration, followed by extraction of the remaining aqueous phase which resulted in reusable solution containing HPCD and buffer components. Each conversion mixture contained freshly harvested cells resuspended to OD=100, 5% (w/v) HPCD and 1% (v/v) isopropanol. The results are shown in Figure 4. A quantitative recovery of HPCD could be shown.

## Claims

1. Process for C-25 selective hydroxylation of vitamin D3 metabolites comprising:
(a) providing a non-hydroxylated form of vitamin D3 as substrate in the presence of a solubilizing agent,
(b) providing a C-25 selective biocatalyst,
(c) contacting the substrate of (a) with the biocatalyst of (b) under suitable conditions for formation of C-25 hydroxylated vitamin D3 metabolites,
(d) isolating and recovering the biocatalyst from step (c) from the other compounds,
(e) repetition of step (c) and (d) for at least 2-3 cycles;
wherein the ability of the biocatalyst towards C-25 hydroxylation is not decreased with each cycle.

2. The process according to claim 1, wherein the solubilizing agent is cyclodextrin, preferably 2-hydroxypropyl-β-cyclodextrin (HPCD).

3. The process according to claim 2, wherein the concentration of HPCD is in the range of 1-15% (w/v).

4. The process according to any one of claims 1 to 3, further comprising recycling or re-using the solubilizing agent.

5. The process according to claim 4, comprising recovering the HPCD at the end of the biotransformation from the liquid phase and separating the HPCD from the vitamin D3 derivatives and/or metabolites.

6. The process according to any one of claims 2 to 5, wherein the HPCD is re-used for at least about 2 cycles, preferably for at least about 2, 3, 4, 5, 6, 7, 8, 9, 10 cycles

7. The process according to claim 6, wherein the degradation of HPCD used in the at least 2 cycles is in the range of 20% or less.

8. The process according to claim 7, wherein the degradation of HPCD is in the range of less than 1%.

9. The process according to any one of claims 4 to 8, wherein the recycling of HPCD is performed under aerobic conditions.

10. The process according to any one of claims 1 to 9, wherein the biocatalyst is used in the form of whole cells or a cell extract.

11. The process according to any one of claims 1 to 10, wherein the biocatalyst is selected from oxygenases, particularly P450 monooxygenases, peroxygenases or members of the iron sulfur molybdoenzyme family, preferably selected from steroid C25 dehydrogenase selectively hydroxylating the C25-position of the substrate.

12. The process according to claim 11, wherein the biocatalyst is selected from O2-sensitive S25DH as originally isolated from *Sterolibacterium denitrificans.*

13. The process according to any one of claims 1 to 11, wherein the substrate is selected from vitamin D3 or 7-dehydrocholesterol (7-DHC).

14. Production of C25-hydroxylated forms of vitamin D3 comprising mixing a suitable substrate with HPCD and optionally EtOH with a biocatalyst, wherein both the biocatalyst as well as the HPCD are recycled or re-used within the process.
